# EUROPEAN PATENT APPLICATION

(11) **EP 4 729 544 A1**
(43) Date of publication of application: **22.04.2026**
(21) Application number: 24823754.7
(22) Date of filing: 14.06.2024
(51) Int. Cl.: C07K 16/28, G01N 33/574, A61P 35/00, G01N 33/68, A61K 39/00

(54) **USE OF CLAUDIN-3 AS TARGET FOR DIAGNOSIS AND TREATMENT OF SMALL CELL LUNG CANCER**

(30) Priority: 15.06.2023 KR 20230077110; 05.01.2024 KR 20240002208
(71) Applicant: Abion Inc., Seoul 08394 (KR)
(72) Inventor: CHOI, Jun Young, Seoul 08394 (KR); KIM, Na Young, Seoul 08394 (KR); LEE, Sae Hyung, Seoul 08394 (KR)
(74) Representative: Biggi, Cristina
(86) International application number: PCT/KR2024/008255
(87) International publication number: WO 2024/258238

(57) **Abstract**

The present invention relates to a use of claudin-3 as a target for treatment of small cell lung cancer, and more specifically to a side effect-reduced composition for prevention or treatment of small cell lung cancer, comprising an anti-claudin-3-specific antibody as an active ingredient. The composition, according to the present invention, does not exhibit ADCC activity in normal cells compared to small cell lung cancer cells, and thus may be usefully employed in the development of a small cell lung cancer-specific therapeutic agent having few side effects.

## Description

### [Technical Field]

The present application claims priority to Korean Patent Application No. 10-2023-0077110, filed on June 15, 2023, and Korean Patent Application No. 10-2024-0002208, filed on January 5, 2024, the entire disclosures of which are incorporated herein by reference. The present disclosure relates to the use of claudin-3 as a target for the diagnosis and treatment of small cell lung cancer, and more specifically, to a composition for preventing or treating small cell lung cancer with reduced side effects, comprising an anti-claudin-3 antibody as an active ingredient.

### [Background Art]

Lung cancer is a leading cause of cancer-related deaths worldwide, accounting for one-fourth of all cancer deaths, and the 5-year survival rate for all lung cancer patients remains very low at only about 15%. Lung cancer is classified into two subtypes: non-small cell lung cancer (NSCLC) and small cell lung cancer (SCLC), with SCLC accounting for approximately 13% of all lung cancers. In particular, SCLC is recognized as the most fatal type of lung cancer due to its limited treatment options and frequent recurrence and metastasis after treatment, with a 5-year survival rate of only 7% for all SCLC patients, indicating a very poor prognosis.

The treatment for SCLC primarily involves platinum-based chemotherapy, etoposide, and checkpoint inhibitors (such as atezolizumab or durvalumab), and recently, lurbinectedin was approved as a treatment for SCLC by the U.S. FDA in 2020.

However, these treatment methods are also used for other types of cancer and cannot be considered specific treatments for SCLC. In the case of lurbinectedin, various side effects such as leukopenia, lymphopenia, fatigue, anemia, dyspnea, vomiting, and cough have been reported. Therefore, there is an urgent need to develop new therapeutic agents that are specific to SCLC while having fewer side effects.

In addition, the diagnosis of SCLC is conducted through medical history and physical examination, imaging tests such as chest X-rays, CT, PET, and MRI, cytological and pathological examinations through biopsy, and tests for measuring tumor markers in the blood, such as NSE and ProGRP. These diagnostic methods confirm the presence of SCLC, determine the stage, and establish an appropriate treatment plan. However, there is currently no diagnostic method related to diagnostic markers for specific treatments for SCLC patients. Therefore, the discovery of new diagnostic biomarkers for targeted therapy for individual SCLC patients is urgently needed.

Meanwhile, claudin proteins are important structural and functional components of epithelial tight junctions located between adjacent cells, playing roles in regulating cell-to-cell permeability, maintaining ion homeostasis, and supporting cell adhesion and polarity. Claudin proteins are transmembrane proteins with a molecular weight of 22 to 27 kDa that form protective barriers by multimerizing within or across the cell membrane. To date, 24 types of claudin proteins have been reported, and they have been identified to exhibit diverse expression patterns in various organs. In particular, claudin proteins, as proteins located in tight junctions, are not exposed externally in normal cells and are maintained between membranes. However, in cancer cells, which lose their structural integrity and exhibit overgrowth during tumorigenesis, tight junctions are lost, leading to the exposure of claudin proteins. This characteristic allows claudin proteins to act as markers for cancer, making them of great interest as cancer-specific markers or therapeutic targets.

### [Technical Problem]

Accordingly, the present inventors have made diligent efforts to develop a specific therapeutic agent for small cell lung cancer with reduced side effects. As a result, it was confirmed that targeting claudin-3 protein exhibits low ADCC (Antibody-dependent cellular cytotoxicity) activity against normal cells, while showing high ADCC activity only against small cell lung cancer cells. Furthermore, it was confirmed that claudin-3 is significantly overexpressed in patients with small cell lung cancer, verifying its potential as a diagnostic biomarker for small cell lung cancer patients, thereby completing the present invention.

Therefore, an object of the present disclosure is to provide a composition for preventing or treating small cell lung cancer with reduced side effects, comprising an anti-claudin-3 antibody as an active ingredient.

Another object of the present disclosure is to provide a composition for preventing or treating small cell lung cancer with reduced side effects, consisting of an anti-claudin-3 antibody.

Yet another object of the present disclosure is to provide a composition for preventing or treating small cell lung cancer with reduced side effects, consisting essentially of an anti-claudin-3 antibody.

Still another object of the present disclosure is to provide a method for providing information for diagnosing small cell lung cancer, comprising measuring the protein level of claudin-3 from a biological sample isolated from a subject.

Another object of the present disclosure is to provide the use of an anti-claudin-3 antibody for the manufacture of a pharmaceutical composition for treating small cell lung cancer with reduced side effects.

Another object of the present disclosure is to provide a method for treating small cell lung cancer with reduced side effects, comprising administering an effective amount of a composition comprising anti-claudin-3 as an active ingredient to a subject in need thereof.

Another object of the present disclosure is to provide a method for treating small cell lung cancer, comprising administering an effective amount of a composition comprising anti-claudin-3 as an active ingredient to a patient with small cell lung cancer who has received chemotherapy drugs.

### [Solution to problem]

To achieve the above objectives, the present disclosure provides a composition for preventing or treating small cell lung cancer with reduced side effects, comprising an anti-claudin-3 antibody as an active ingredient.

To achieve another objective of the present disclosure, the present disclosure provides a composition for preventing or treating small cell lung cancer with reduced side effects, consisting of an anti-claudin-3 antibody.

To achieve yet another objective of the present disclosure, the present disclosure provides a composition for preventing or treating small cell lung cancer with reduced side effects, consisting essentially of an anti-claudin-3 antibody.

To achieve yet another objective of the present disclosure, the present disclosure provides a method for providing information for diagnosing small cell lung cancer, comprising measuring the level of claudin-3 protein from a sample of a patient suspected of having small cell lung cancer.

To achieve yet another objective of the present disclosure, the present disclosure provides the use of an anti-claudin-3 antibody for the manufacture of a pharmaceutical composition for treating small cell lung cancer with reduced side effects.

To achieve yet another objective of the present disclosure, the present disclosure provides a method for treating small cell lung cancer with reduced side effects, comprising administering an effective amount of a composition comprising an anti-claudin-3 as an active ingredient to a subject in need thereof.

To achieve yet another objective of the present disclosure, the present disclosure provides a method for treating small cell lung cancer, comprising administering an effective amount of a composition comprising an anti-claudin-3 as an active ingredient to a patient with small cell lung cancer who has received chemotherapy drugs.

The present disclosure will now be described in detail.

The present disclosure provides a composition for preventing or treating small cell lung cancer with reduced side effects, comprising an anti-claudin-3 antibody as an active ingredient.

In the present specification, the term "comprising" is used in the same sense as "including" or "characterized by" and does not exclude additional components or steps not specifically mentioned in the composition or method according to the present disclosure. Furthermore, the term "consisting of" means excluding additional elements, steps, or components not specifically described. The term "consisting essentially of" means that the composition or method may include additional components or steps that do not substantially affect the basic and novel characteristics of the described components or steps.

The inventors of the present disclosure, while conducting research on small cell lung cancer, confirmed that the mRNA and protein of claudin-3 are expressed at significantly high levels in small cell lung cancer cell lines. Through this, it was confirmed that claudin-3 protein is a biomarker for diagnosis and treatment of small cell lung cancer. Using an antibody targeting claudin-3 protein, the antibody-dependent cellular cytotoxicity (ADCC) activity against small cell lung cancer was confirmed, and it was found to exhibit effective anticancer activity. Particularly, even when claudin-3 is expressed in normal cells, ADCC activity is not well observed, whereas in small cell lung cancer, ADCC activity is well observed even at low levels of claudin-3 expression. Specifically, according to one embodiment of the present disclosure, when a high dose of anti-claudin-3 antibody was treated to cancer cell lines and normal cell lines expressing claudin-3, ADCC activity of 30% to 80% was observed in cancer cells, whereas ADCC activity of 7.9% or less or no ADCC cytotoxicity was observed in normal cell lines. This suggests that cytotoxicity is unlikely to occur in normal tissue cells.

This finding indicates that claudin-3 has potential as a diagnostic biomarker for small cell lung cancer and that using an anti-claudin-3 antibody as a therapeutic agent for small cell lung cancer results in fewer side effects. Most anticancer drugs are designed to act on rapidly proliferating cells, as cancer cells are characterized by rapid proliferation and division. However, there are normal cells in the body, such as gastrointestinal mucosal cells, bone marrow cells, hair follicle cells, oral epithelial cells, and reproductive cells, that also proliferate rapidly like cancer cells. These normal cells are affected and destroyed by anticancer drugs, leading to side effects such as leukopenia, lymphopenia, fatigue, anemia, dyspnea, vomiting, stomatitis, cough, and/or reproductive dysfunction. In contrast, the composition according to the present disclosure, comprising an anti-claudin-3 antibody as an active ingredient, does not exhibit ADCC activity in any normal cells in the body that express claudin-3 but exhibits ADCC activity only in small cell lung cancer cells expressing claudin-3, thereby significantly reducing side effects. This constitutes a significant advantage.

Due to this advantage, the composition according to the present disclosure can be administered in much higher doses compared to the known dosage and administration methods of existing anticancer drugs, thereby achieving relatively higher anticancer effects compared to existing anticancer drugs. For example, among monoclonal antibody therapeutics known in the art, the drug dosage for antibodies targeting antigens expressed in normal cells is as follows: Rituximab is administered at 375 mg/m² (non-Hodgkin's lymphoma); trastuzumab is administered at 8 mg/kg (approximately 240 mg/m²) as an initial loading dose, followed by 6 mg/kg (approximately 180 mg/m²) (breast cancer, gastric cancer); bevacizumab is administered at 5 mg/kg (approximately 150 mg/m²) or 10 mg/kg (approximately 300 mg/m²) (metastatic colorectal cancer), 15 mg/kg (approximately 450 mg/m²) (non-small cell lung cancer), 10 mg/kg (approximately 300 mg/m²) (renal cell carcinoma); cetuximab is administered at 400 mg/m² as an initial loading dose, followed by 250 mg/m² (metastatic colorectal cancer, head and neck squamous cell carcinoma). For monoclonal antibody drugs targeting claudin-18.2, the clinical phase 2 dosage is known to be 800 mg/m² as an initial loading dose, followed by 600 mg/m² for zolbetuximab; 10 mg/kg (approximately 300 mg/m²) for TST001; 800 mg/m² for ASKB589; and 20 mg/kg (approximately 800 mg/m²) for BIL93. Considering these facts, the composition according to the present disclosure can be administered at doses of 500 mg/m², 600 mg/m², 700 mg/m², 800 mg/m², 900 mg/m², 1000 mg/m², 1100 mg/m², 1200 mg/m², or 500 mg/m² to 1200 mg/m², 600 mg/m² to 1200 mg/m², 700 mg/m² to 1200 mg/m², 800 mg/m² to 1200 mg/m², 900 mg/m² to 1200 mg/m², 1000 mg/m² to 1200 mg/m², 1100 mg/m² to 1200 mg/m², or higher, but is not limited thereto. Due to the low side effects of the composition according to the present disclosure, a person skilled in the art can appropriately select and administer the composition according to the present disclosure at higher doses than the dosages of monoclonal antibody drugs targeting claudin known in the art, thereby achieving superior anticancer effects compared to other monoclonal antibody drugs.

In the present disclosure, the "claudin-3 (CLDN3)" protein is a protein belonging to the claudin family, which is present at tight junctions and plays a unique role in eliminating the space between cells at tight junctions. Tight junctions are robust structures that connect adjacent cell membranes in tissues of organisms such as animals. Claudin-3 regulates intercellular permeability to small solutes such as ions, has a molecular weight of 20-34 kDa, and is known as the most important backbone protein of tight junctions with two extracellular loops and four transmembrane structural domains. The claudin-3 protein functions as a barrier-forming protein in barrier such as the blood-brain barrier and intestinal barrier and is also known to be involved in regulating cell proliferation, migration, differentiation, and polarity.

In the present disclosure, the term "antibody" refers to a protein, also called immunoglobulin (Ig), that selectively acts on antigens and is involved in the immune response of living organisms. Natural whole antibodies are generally composed of two pairs of polypeptides-light chains (LC) and heavy chains (HC)-each comprising, multiple domains, and the structure formed by these LC/HC pairs serves as the basic unit of the antibody. The heavy chains of mammalian antibodies are classified into five types, represented by the Greek letters α, β, γ, δ, and µ, which form different types of antibodies such as IgA, IgD, IgE, IgG, and IgM, respectively. Mammalian antibody light chains are classified into two types, λ and κ.

The heavy and light chains of antibodies are structurally divided into variable regions and constant regions based on the variability of their amino acid sequences. The constant region of the heavy chain consists of three or four constant regions, CH1, CH2, and CH3 (for IgA, IgD, and IgG antibodies) or CH4 (for IgE and IgM antibodies), depending on the type of antibody, while the light chain consists of one constant region, LC. The heavy and light chains are aligned side by side, with their variable and constant regions connected by a single covalent disulfide bond. A light chain and the variable regions of the heavy and light chains specifically bind to antigens, and the whole antibody consists of two pairs of heavy and light chains (HC/LC), allowing one molecule of the whole antibody to bind to two identical antigens through its two variable regions, thereby exhibiting bivalent single specificity. The variable region of the antibody that binds to the antigen is called the antigen-binding site, and the part of the antigen recognized by the antibody is called the epitope.

The variable region of the antibody containing the antigen-binding site is subdivided into framework regions (FR) with low sequence variability and hypervariable regions, also known as complementarity-determining regions (CDR), with high sequence variability. VH and VL each contain two CDRs and four FRs arranged in the order FR1-CDR1-FR2-CDR2-FR3-CDR3-FR4 from the N-terminus to the C-terminus. Among the variable regions of the antibody, the CDR, which has the highest sequence variability, directly binds to the antigen and is most important for the antigen specificity of the antibody.

The antibody or fragment thereof according to the present disclosure is not limited in type as long as it has the above-described CDR, VH and VL, or the configuration of the light and heavy chains. The antibody may be in the form of IgG, IgA, IgM, IgE, or IgD, and is preferably an IgG antibody. The IgG may include its subtypes such as IgG1, IgG2, IgG3, and IgG4, but is not limited thereto. The antibody may be a monoclonal antibody derived from a single B cell or a polyclonal antibody derived from multiple B cells, but is preferably a monoclonal antibody, which is a population of antibodies with substantially identical amino acid sequences in the heavy and light chains. The antibody or fragment thereof according to the present disclosure may be conjugated with enzymes, fluorescent substances, radioactive substances, proteins, or the like, but is not limited thereto.

The antibody according to the present disclosure may be derived from any animal, including mammals and birds, including humans, and is preferably derived from humans or may be a chimeric antibody comprising a portion of an antibody derived from humans and a portion of an antibody derived from another species.

In addition, the antibody fragment in the present disclosure refers to a fragment that retains the antigen-specific binding ability of the whole antibody and may specifically be in the form of Fab, F(ab'), F(ab')2, Fv, scFv, diabody, dsFv or the like.

Fab (fragment antigen-binding) is an antigen-binding fragment of an antibody, consisting of one variable domain and one constant domain of each of the heavy and light chains. F(ab')2 is a fragment generated by pepsin digestion of an antibody, in which two Fab fragments remain linked through inter-heavy-chain disulfide bond in the hinge region. F(ab') is a monomeric antibody fragment obtained by reducing the disulfide bond of the F(ab')2 fragment, resulting in a Fab fragment that retains a portion of the heavy-chain hinge region. Fv (variable fragment) is an antibody fragment consisting only of the variable regions of the heavy and light chains. scFv (single chain variable fragment) is a recombinant antibody fragment in which the variable regions of the heavy chain (VH) and light chain (VL) are connected by a flexible peptide linker. Diabody is an antibody fragment in which the VH and VL domains of a scFv are connected by a very short linker that preventing intrachain pairing, causing each VH and VL to pair instead with the complementary domain of another scFv of the same format, thereby forming a dimeric structure. dsFv refers to a polypeptide in which one amino acid residue of each of the VH and VL is substituted with a cysteine residue, and the cysteine residues are connected via S-S bonds. The amino acid residues to be substituted with cysteine residues can be selected based on the prediction of the antibody's three-dimensional structure according to methods known in the art, such as those described by Reiter et al.

In one embodiment of the present disclosure, the antibody according to the present disclosure utilizes the antibody disclosed in the applicant's prior registered patent No. 10-2340989. Specifically, the antibody or fragment thereof according to the present disclosure may be an antibody comprising an antibody heavy chain variable region (VH) comprising a heavy chain complementarity-determining region (VH-CDR1) comprising the amino acid sequence represented by SEQ ID NO: 1, a heavy chain complementarity-determining region (VH-CDR2) comprising the amino acid sequence represented by SEQ ID NO: 2, and a heavy chain complementarity-determining region (VH-CDR3) comprising the amino acid sequence represented by SEQ ID NO: 3; and an antibody light chain variable region (VL) comprising a light chain complementarity-determining region (VL-CDR1) comprising the amino acid sequence represented by SEQ ID NO: 4, a light chain complementarity-determining region (VL-CDR2) comprising the amino acid sequence represented by SEQ ID NO: 5, and a light chain complementarity-determining region (VL-CDR3) comprising the amino acid sequence represented by SEQ ID NO: 6.

However, as described above, the feature of the present disclosure that claudin-3 protein is an effective target for small cell lung cancer is not due to any specific antibody targeting claudin-3 but is attributed to the characteristics of the claudin-3 protein itself in small cell lung cancer. Therefore, the effects can be equally obtained with any anti-claudin-3 antibody protein, not limited to the antibodies having the specific complementarity-determining region sequences described above.

In addition, in some embodiments, the antibody according to the present disclosure may be chemically modified (e.g., with one or more moieties attached to the antibody), glycosylated (e.g., hypofucosylated, afucosylated, or with increased sialylation), or modified or produced under cell line and/or cell culture conditions to alter one or more functional characteristics of the antibody.

In some embodiments, the antibody according to the present disclosure includes an Fc region with modified glycosylation to enhance ADCC activity. Specifically, in some embodiments, the Fc region of the antibody of the present disclosure may not contain fucose sugars. The afucosylated antibody may be produced using cell lines expressing heterologous enzymes that deplete the intracellular fucose pool or using host cell lines with a deleted endogenous α-1,6-fucosyltransferase (FUT8) gene, but is not limited thereto.

In one embodiment of the present disclosure, ADCC activity was measured using an anti-claudin-3 and anti-CD3 bispecific antibody, and it was confirmed that excellent anticancer activity was exhibited in all tested cell lines. Therefore, the antibody according to the present disclosure may be a bispecific antibody. The term "bispecific antibody" refers to an antibody having two binding sites specific for different antigens. The antibody of the present disclosure may be a bispecific antibody that specifically binds to CD3 in addition to claudin-3 described above, but is not limited thereto. In addition to CD3, the antibody may also be applied without limitation to other targets known to be applicable to small cell lung cancer, such as DLL3, B7H3, PD-L1, IGFR, VEGFR, FGFR, TROP2, and SEZ6.

In one embodiment of the present disclosure, it was confirmed that the expression level of claudin-3 protein increased when chemotherapy drugs were administered to small cell lung cancer cell lines. This indicates that in the case of antibody-based drugs targeting claudin-3 protein, such as the composition according to the present disclosure, the binding opportunity increases, thereby exhibiting superior anticancer activity. Therefore, the composition for treating small cell lung cancer according to the present disclosure is characterized by being administered to patients with small cell lung cancer who have received chemotherapy drugs.

In the present disclosure, the chemotherapy drug may be used without limitation as long as it is applicable to patients with small cell lung cancer, but is preferably cisplatin, carboplatin, etoposide, topotecan, cyclophosphamide, doxorubicin, vincristine, lurbinectedin, irinotecan, or a combination thereof.

In the present disclosure, the content of the composition is not significantly limited depending on the purpose or aspect of use and may, for example, be 0.01 to 99% by weight, preferably 0.5 to 50% by weight, and more preferably 1 to 30% by weight, based on the total weight of the composition. Additionally, the pharmaceutical composition according to the present disclosure may further comprise additives such as pharmaceutically acceptable carriers, excipients, or diluents in addition to the active ingredient.

The present disclosure also provides a method for providing information for diagnosing small cell lung cancer, comprising measuring the level of claudin-3 protein from a sample of a patient suspected of having small cell lung cancer.

In the present disclosure, the method for providing information for diagnosing small cell lung cancer may further comprise comparing the expression level of claudin-3 protein or the gene encoding it in a sample isolated from a patient suspected of having small cell lung cancer with that of a healthy individual after the above step.

In the method according to the present disclosure, the biological sample may be derived from a specific tissue or organ of the patient and is preferably derived from lung tissue. More specifically, the biological sample may include fluid samples, including blood, serum, plasma, lymph, breast milk, urine, feces, and saliva, but is not limited thereto.

In the method according to the present disclosure, the claudin-3 expression level can be measured by one or more methods selected from the group consisting of reverse transcription-polymerase chain reaction (RT-PCR), enzyme-linked immunosorbent assay (ELISA), radioimmunoassay, immunohistochemistry, microarray, western blotting, exosome, circulating tumor cells, and flow cytometry, but the method is not limited thereto. A person skilled in the art can perform the method according to the present disclosure without limitation by using any method known in the art that can measure the expression level of claudin-3 from a biological sample.

In addition, the present disclosure provides the use of an anti-claudin-3 antibody for the manufacture of a pharmaceutical composition for treating small cell lung cancer with reduced side effects.

The term "effective amount" in the present disclosure refers to an amount that, when administered to a subject, exhibits an effect of improving, treating, detecting, diagnosing, or suppressing or reducing small cell lung cancer, and the term "subject" may refer to an animal, preferably a mammal, particularly an animal including a human, and may also include cells, tissues, organs and the like derived from an animal.

The term "treatment" in the present disclosure comprehensively refers to ameliorating symptoms arising from small cell lung cancer, and may include curing the disease, substantially preventing the disease, or improving the condition of the disease, and includes alleviating, curing, or preventing one or the majority of symptoms resulting from the disease, but is not limited thereto.

In addition, the present disclosure provides a method for treating small cell lung cancer with reduced side effects, comprising administering an effective amount of a composition comprising an anti-claudin-3 as an active ingredient to a subject in need thereof.

In addition, the present disclosure provides a method for treating small cell lung cancer, comprising administering an effective amount of a composition comprising an anti-claudin-3 as an active ingredient to a patient with small cell lung cancer who has received chemotherapy drugs.

The "subject" may be a patient with small cell lung cancer who has received chemotherapy drugs, and the chemotherapy drug may be cisplatin, carboplatin, etoposide, topotecan, cyclophosphamide, doxorubicin, vincristine, lurbinectedin, irinotecan, or a combination thereof.

In addition, the present disclosure provides a method for treating small cell lung cancer, comprising administering an effective amount of a composition comprising an anti-claudin-3 as an active ingredient to a patient with small cell lung cancer who has received chemotherapy drugs.

### [Advantageous Effects of Invention]

The present disclosure relates to a composition for preventing or treating small cell lung cancer with reduced side effects, comprising an anti-claudin-3 antibody as an active ingredient. When targeting claudin-3, the ADCC activity of the antibody is significantly higher in cancer cells while being lower in normal cells, and thus, it can be usefully employed in the development of a small cell lung cancer-specific therapeutic agent with fewer side effects.

### [Brief Description of the Drawings]

Fig. 1a to Fig. 1c show that the mRNA of claudin-3 is expressed at the highest rate in small cell lung cancer among various cancer types.
Fig. 2 shows the results of analyzing the correlation between the levels of claudin-3 protein and mRNA.
Fig. 3 shows the results of analyzing the expression rate of claudin-3 protein in small cell lung cancer cell lines using flow cytometry.
Fig. 4a to Fig. 4d show the results of analyzing the expression rate of claudin-3 protein in claudin-3-positive small cell lung cancer cell lines using immunohistochemistry.
Fig. 5a to Fig. 5i show the results of confirming the anticancer effect of a claudin-3-targeting monoclonal antibody in claudin-3-positive small cell lung cancer cell lines using the LDH method.
Fig. 6a to Fig. 6f show the results of confirming the anticancer effect of a claudin-3-targeting monoclonal antibody in claudin-3-positive normal cell lines using the LDH method.
Fig. 7 shows the results of comparing the expression levels of claudin-3 and β-actin when the chemotherapy drugs etoposide, cisplatin, and carboplatin were each treated in small cell lung cancer cell lines.
Fig. 8a to Fig. 8c show the results of confirming the anticancer effect of an anti-claudin-3 and anti-CD3 bispecific antibody in claudin-3-positive small cell lung cancer cell lines using the WST method.

### [Detailed Description of the Invention]

Hereinafter, the present disclosure will be described in detail. However, the following examples are merely illustrative of the present disclosure, and the content of the present disclosure is not limited to the following examples.

### Example 1. Analysis of Overexpression of Claudin-3 (Claudin 3) mRNA in Small Cell Lung Cancer (SCLC)

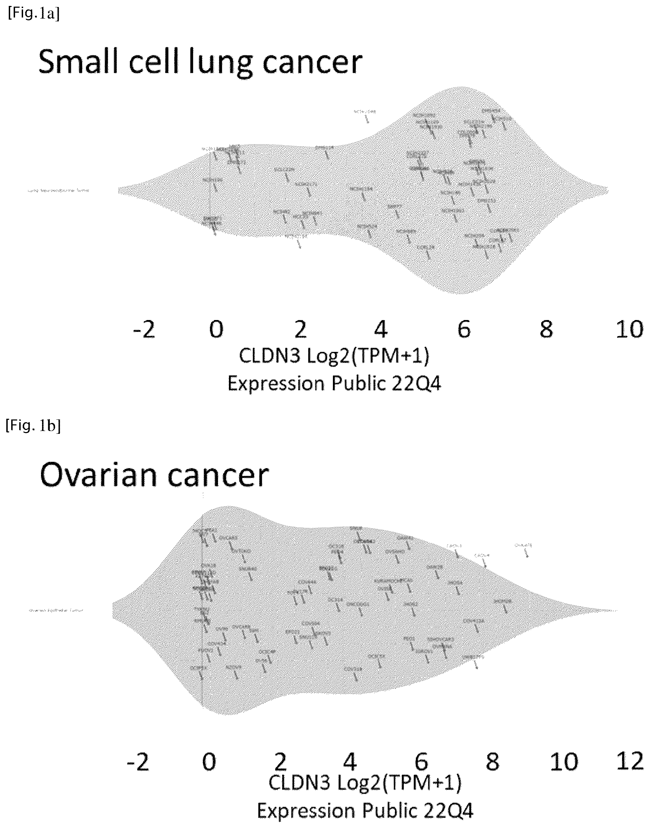

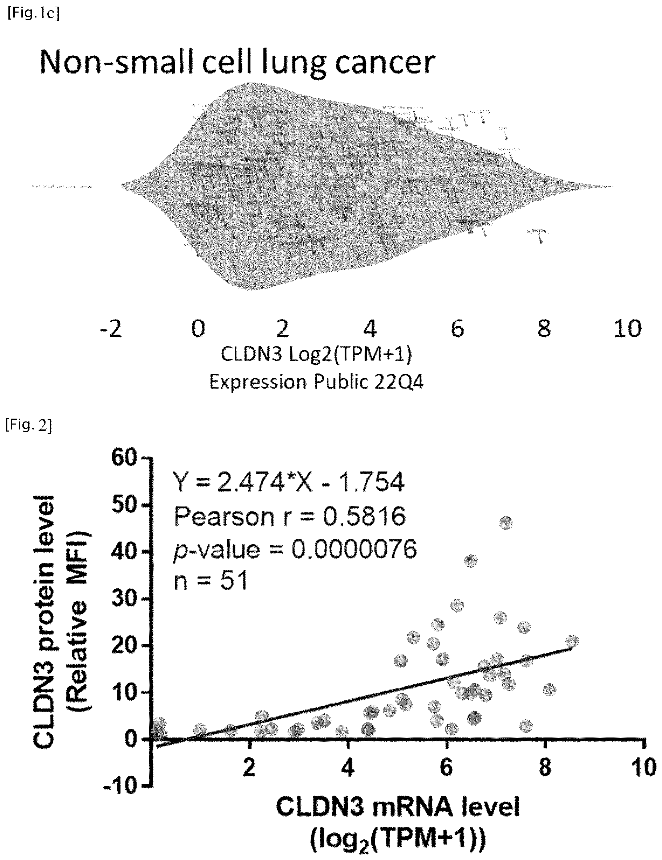

The distribution of claudin-3 mRNA levels in cancer cells of various cancer types was compared using the DepMab database. As a result, it was confirmed that a large number of cells with high levels of claudin-3 mRNA specifically exist in small cell lung cancer (see Figures 1a to 1c).

### Example 2. Analysis of Correlation Between Claudin-3 Protein and mRNA

Considering that the mRNA expression level of a specific gene does not always match the protein expression level, the correlation between claudin-3 protein and mRNA expression was analyzed in 51 cell lines. For this purpose, information on mRNA expression rates of 51 cancer cell lines was obtained from the DepMab database, and the expression rate of claudin-3 protein in these cell lines was confirmed using flow cytometry according to the following method.

Each of the 51 cell lines was cultured and harvested, and the cells were adjusted to a density of 2 x 10⁶ cells/mL (100 µl). The cells were then incubated with 10 µg/mL of anti-claudin-3 antibody (the antibody identified using claudin-3-overexpressing cell lines) for 45 minutes at 4°C, followed by washing three times with PBS/1% FBS buffer. Subsequently, the cells were treated with anti-human IgG-FITC antibody (Jackson Immunoresearch Laboratories) diluted 1:100 for 45 minutes at 4°C, and washed three times with PBS/1% FBS buffer. The samples were analyzed using a flow cytometer (BD FACSCalibur). The correlation between claudin-3 protein and mRNA expression rates was evaluated using the Pearson correlation coefficient. As a result, it was confirmed that there is a positive correlation between claudin-3 protein and mRNA expression rates with r = 0.5816 (see Figure 2).

### Example 3. Analysis of Claudin-3 Protein Expression Levels in Small Cell Lung Cancer

The expression of claudin-3 protein was evaluated using flow cytometry in 16 small cell lung cancer cell lines (Type A: NCI-H209, NCI-H69, NCI-H1688, NCI-H187, NCI-H146, NCI-H719, NCI-H889; Type A/N: SCLC-21H, COR-L279, DMS 53; Type N: COR-L24, NCI-H417; Type P: NCI-H526, COR-L311; Type Y: NCI-H2286, SW1271). The specific method for evaluating protein expression levels was performed in the same manner as in Example 2.

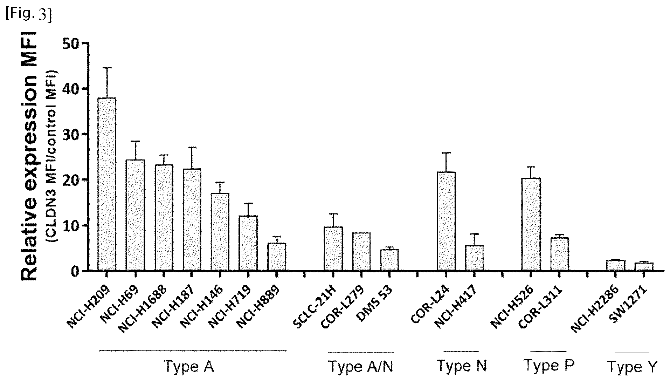

As a result, as shown in Figure 3, claudin-3 positivity was confirmed in all 16 cell lines used in the analysis, and it was particularly confirmed that claudin-3 is highly expressed in all subtypes of small cell lung cancer except for Type Y small cell lung cancer.

### Example 4. Analysis of Claudin-3 Protein Expression Levels in Claudin-3 Positive Small Cell Lung Cancer Cell Lines

Among the claudin-3 positive cell lines identified in Example 3, cell lines exhibiting high, medium, and low levels of claudin-3 protein expression were selected, and the expression levels of claudin-3 protein were evaluated through immunohistochemistry.

First, based on the results of Example 3 and Figure 3, H209, H146, and H889 cell lines were selected as cell lines exhibiting high, medium, and low levels of claudin-3 protein expression, respectively. Each cell line was cultured and harvested and FFPE blocks were prepared using each cell line. The prepared FFPE blocks were subjected to immunohistochemistry to determine the expression levels of claudin-3 protein.

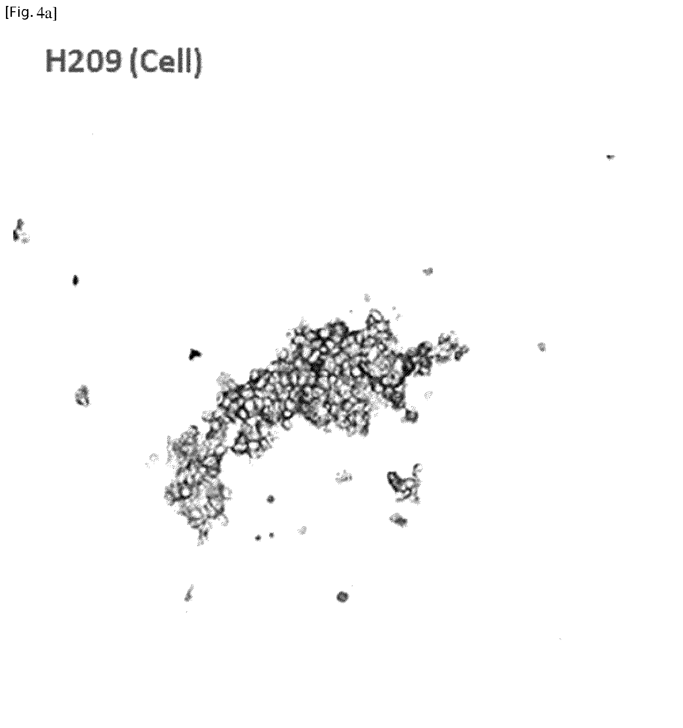

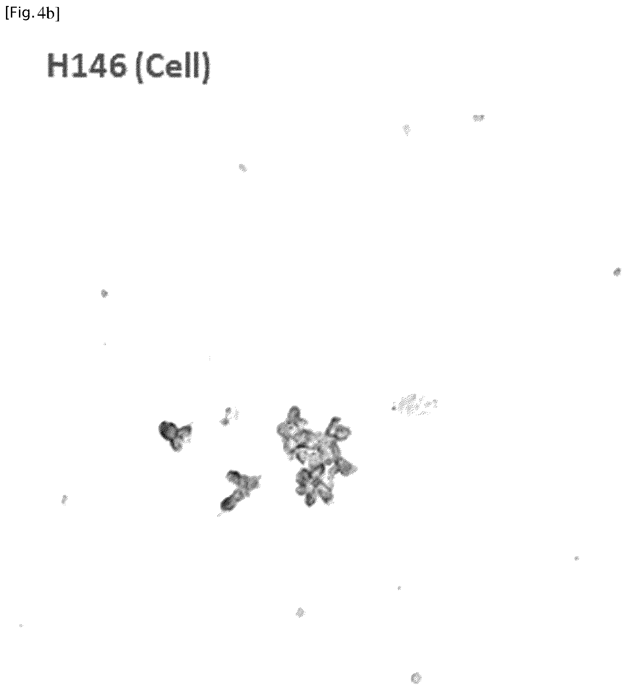

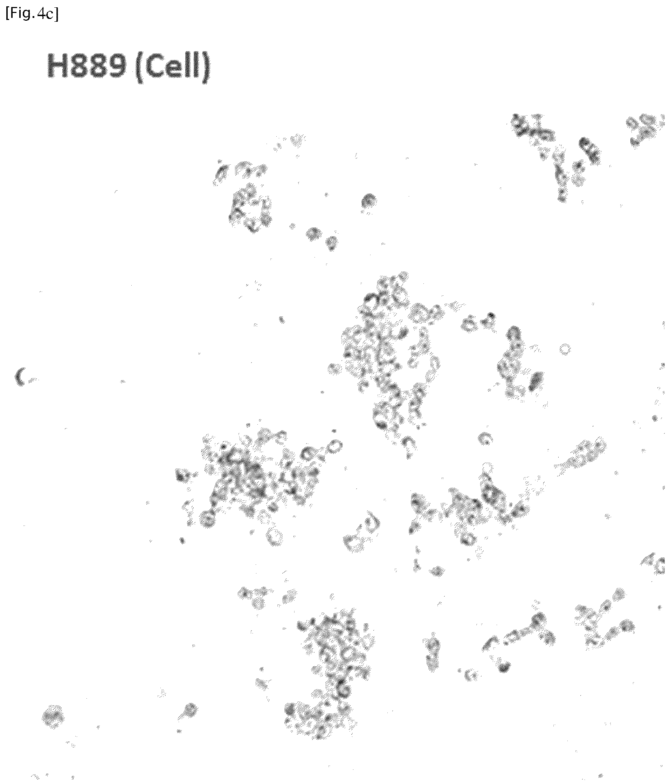

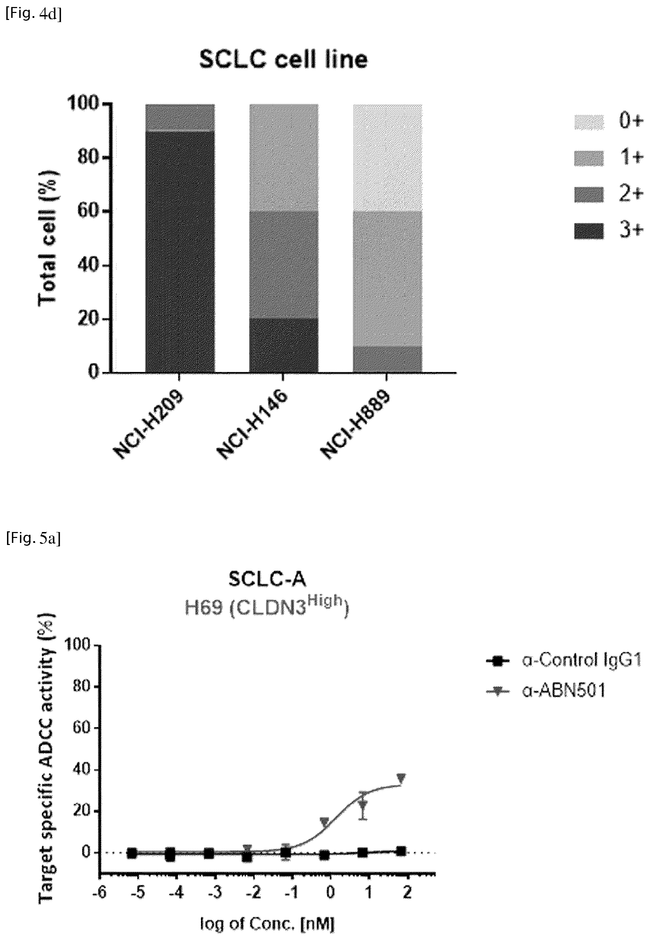

As a result, as shown in Figures 4a to 4d, even in cell lines that exhibited relatively low claudin-3 protein expression as determined by flow cytometry, immunohistochemistry analysis showed expression levels of 2+ and 1+, indicating at least moderate levels of claudin-3 expression.

### Example 5. Confirmation of Anticancer Activity of Monoclonal Antibody Targeting Claudin-3 in Claudin-3 Positive Small Cell Lung Cancer Cell Lines

The anticancer activity was confirmed in claudin-3 positive small cell lung cancer cell lines using an anti-claudin-3 monoclonal antibody (aABN501, afucosylated) and NK92-MI-CD16a cells expressing human CD16a.

Detailed information on the ABN501 antibody used in this example can be found in Korea Patent No. 10-2340989, and more specifically, the complementarity determining region (CDR) sequences of the antibody are as follows.

**[Table 1]**

| Region | Amino acid sequence | SEQ ID NO. |
|---|---|---|
| VH-CDR1 | SYAMS | 1 |
| VH-CDR2 | I INPSGASTSHAQRFQ | 2 |
| VH-CDR3 | RYGRYGSFDI | 3 |
| VL-CDR1 | SGSTSNIGRNYVS | 4 |
| VL-CDR2 | DTSNKHF | 5 |
| VL-CDR3 | QSYDSSKVV | 6 |

Each cell line was seeded at a density of 1x10⁶ cells/mL (50 µl) into a 96-well plate. The cells were treated with an anti-claudin-3 antibody at concentrations ranging from 10 µg/mL to 0.001 ng/mL at room temperature for 30 minutes. Then, NK92-MI-CD16a cells were added at a density of 4x10⁶ cells/mL (25 µl), and incubated for 4 hours. Thereafter, anticancer activity was confirmed using an LDH (lactate dehydrogenase) assay.

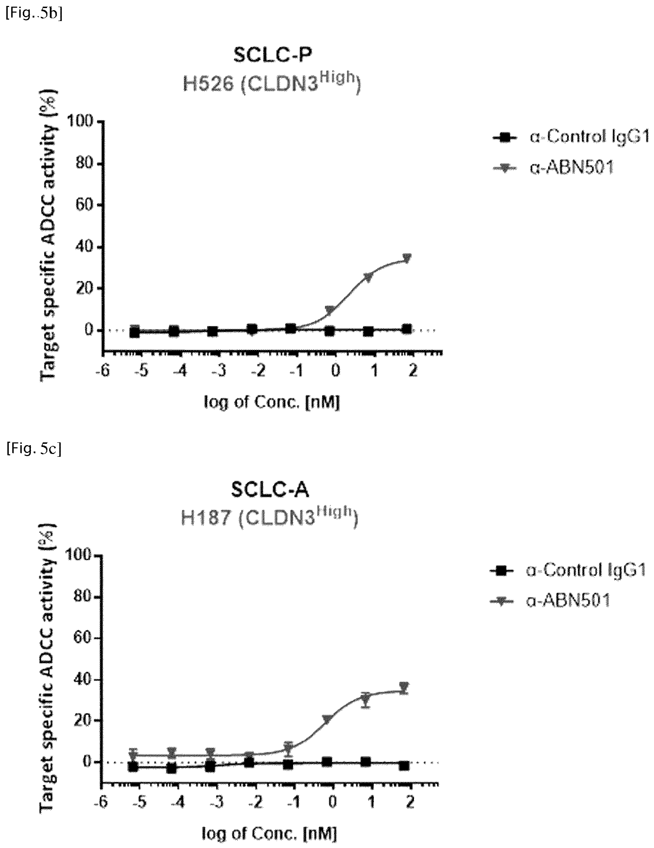

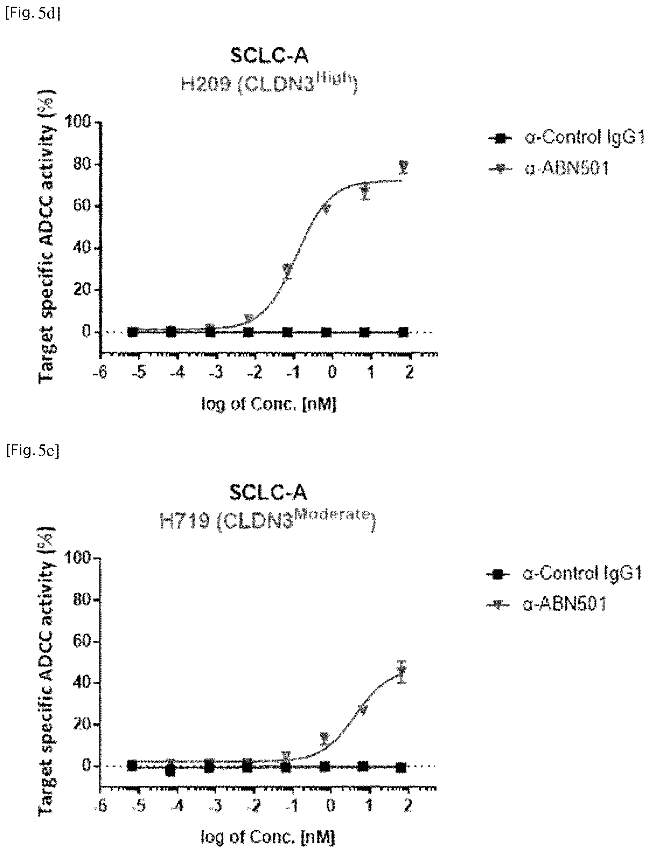

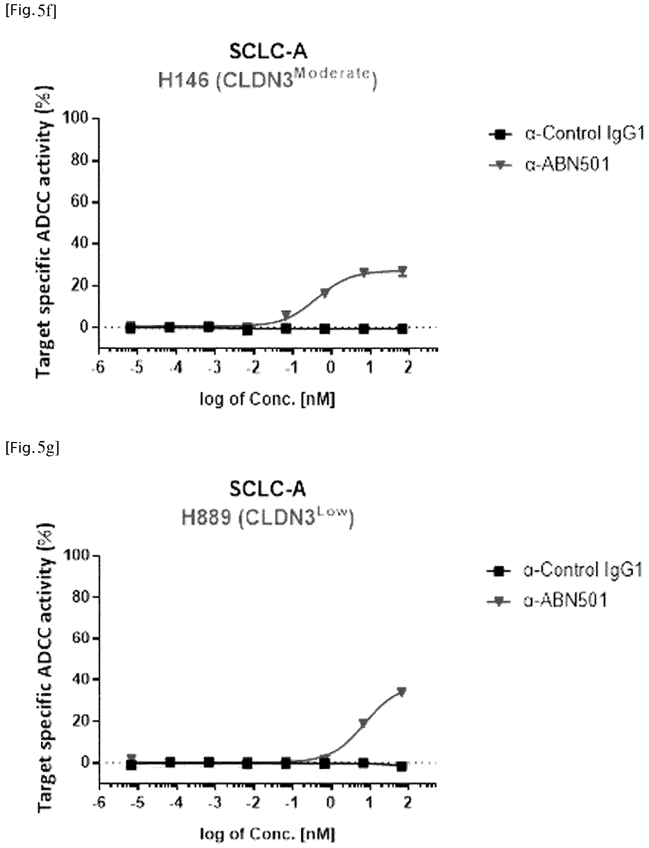

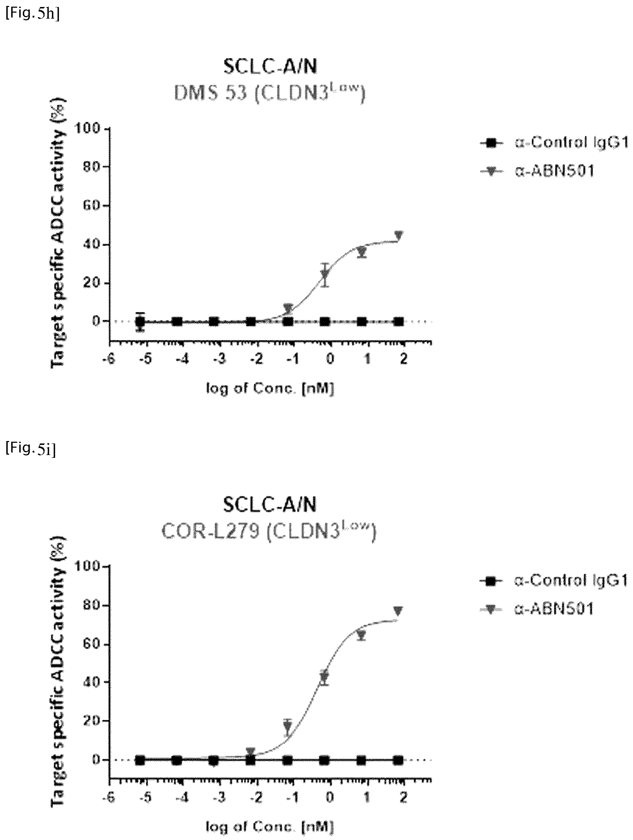

As a result, as shown in Figures 5a to 5i, effective anticancer activity was confirmed in all claudin-3 positive cell lines.

### Example 6. Confirmation of Anticancer Activity of Monoclonal Antibody Targeting Claudin-3 in Claudin-3 Positive Normal Cell Lines

The anticancer activity was confirmed in normal cell lines expressing claudin-3 using an anti-claudin-3 monoclonal antibody (aABN501, afucosylated) and NK92-MI-CD16a cells expressing human CD16a.

Each cell line was seeded at a density of 2x10⁵ cells/mL (100 µl) into a 96-well plate and cultured until reaching 100% confluency. After removing the culture medium, the cells were treated with an anti-claudin-3 antibody at concentrations ranging from 10 µg/mL to 0.001 ng/mL (final concentration) (75 µl) at room temperature for 30 minutes, followed by the addition of NK92-MI-CD16a cells at a density of 4x10⁶ cells/mL (25 µl), and incubated for 4 hours. Toxicity was then confirmed using an LDH (lactate dehydrogenase) assay.

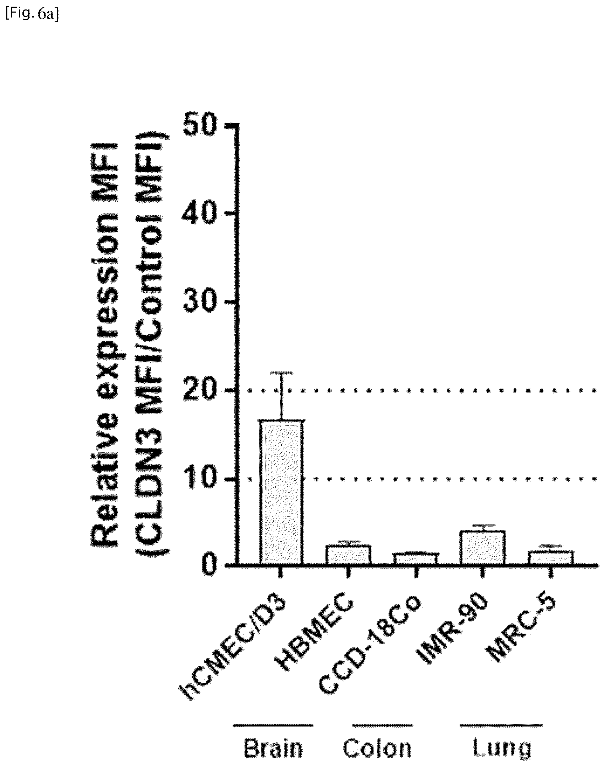

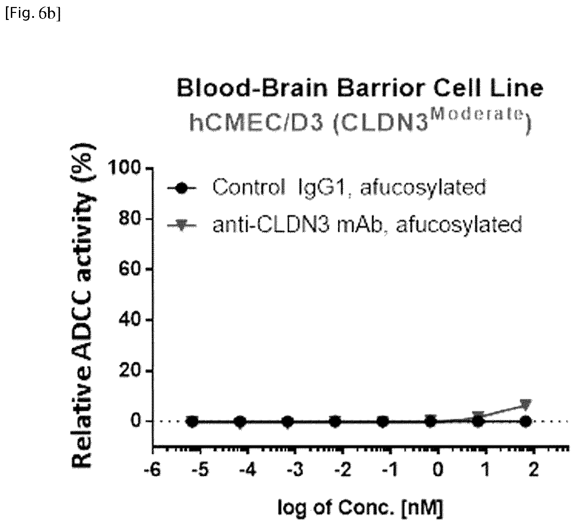

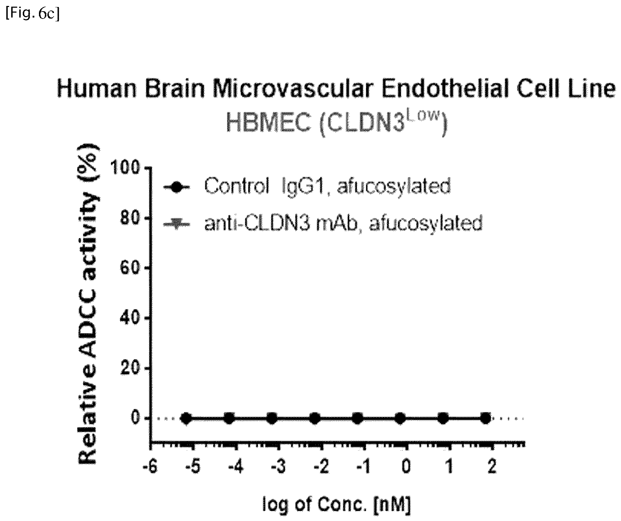

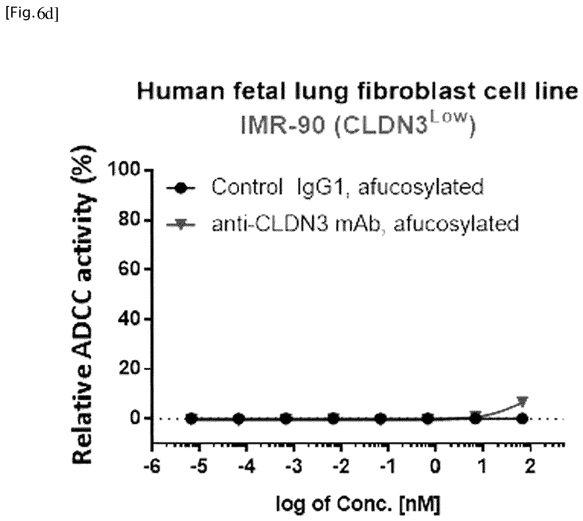

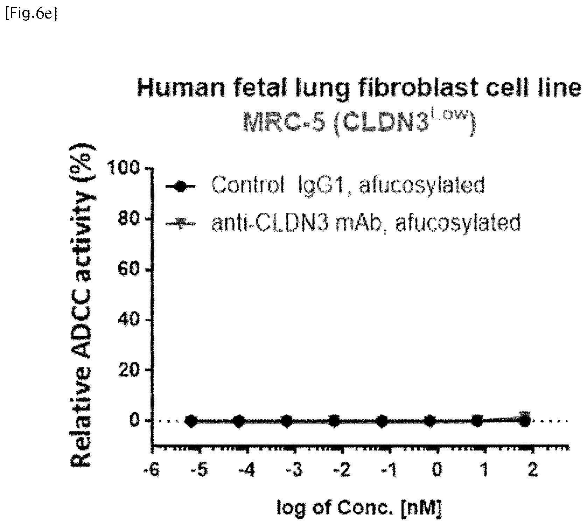

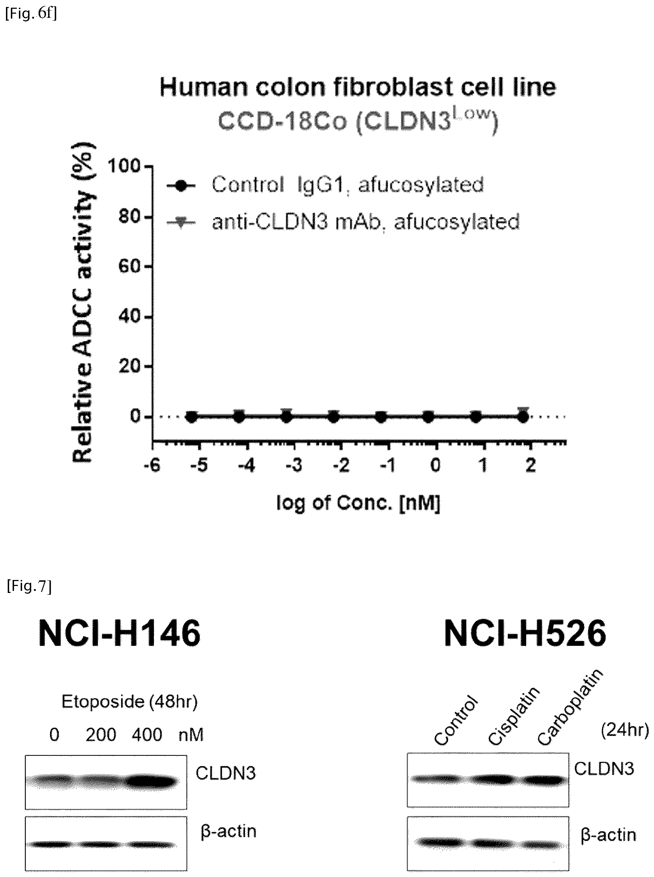

As a result, as shown in Figures 6a to 6f, it was confirmed that claudin-3 positive normal cells did not induce cell death.

Comparing these results with Example 5 and Figures 5a to 5i, when treated with a high dose of 66.6 nM (10 µg/mL) of the antibody, ADCC activity of 30% to 80% was observed in cancer cell lines, although it varied depending on the type of cell line and claudin-3 expression rate. In contrast, normal cells exhibited low ADCC activity of 7.9% or less. This value observed in normal cells corresponds to a value appearing at a dose on average more than 100-fold lower even when compared to small cell lung cancer cell lines having low claudin-3 expression rates.

### Example 7. Increase in Claudin-3 Expression Rate by Chemotherapy in Small Cell Lung Cancer

NCI-H146 and NCI-H526 small cell lung cancer cell lines were seeded at a density of 1x10⁶ cells into 100π culture dishes and treated with etoposide (200, 400 nM), cisplatin (5 µM), or carboplatin (100 µM), which are chemotherapy drugs for small cell lung cancer. The cells were then cultured for 48 hours or 24 hours, respectively, after which the cells were harvested and cell lysates were obtained. Subsequently, the expression levels of claudin-3 and β-actin were analyzed using Western blotting.

As a result, as shown in Figure 7, it was confirmed that the expression level of claudin-3 protein increased in small cell lung cancer cell lines due to chemotherapy. This indicates that the binding opportunity to antibody-based drugs targeting claudin-3 increases, which may contribute to enhanced anticancer activity.

### Example 8. Confirmation of Anticancer Activity of Claudin-3 Targeting CD3 Bispecific Antibody in Claudin-3 Positive Small Cell Lung Cancer Cell Lines

The anticancer activity of an anti-claudin-3 x CD3 bispecific antibody was confirmed in claudin-3 positive small cell lung cancer cell lines using human PBMC. Each cell line was seeded at a density of 4x10⁵ cells/mL (50 µl) into a U-shaped 96-well plate, preincubated with the anti-claudin-3 x CD3 bispecific antibody at concentrations ranging from 0.1 pM to 1 µM of anti-claudin-3 x CD3 bispecific antibody in a 37°C, 5% CO₂ incubator for 30 minutes. Subsequently, hPBMCs (Lot No. 238230218-C) werer added at a density of 4x10⁶ cells/mL (50 µl), and the mixture was cultured for 72 hours. The anticancer activity was then confirmed using the WST assay(DoGENBio, EZ-Cytox).

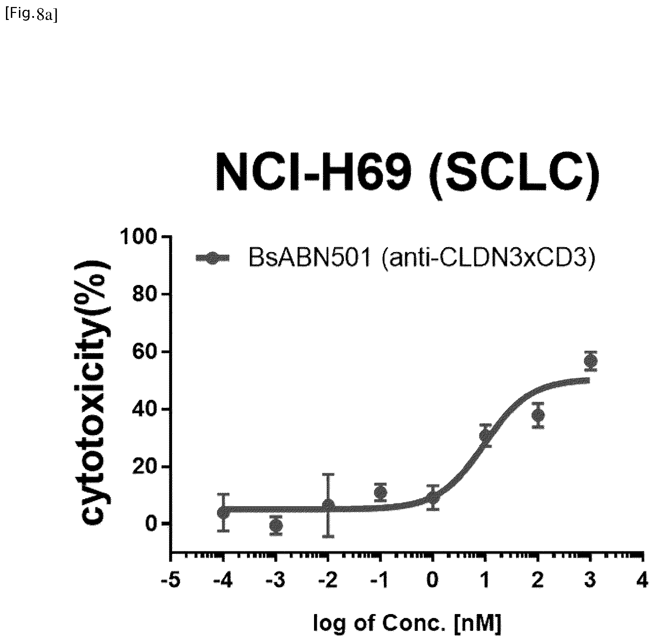

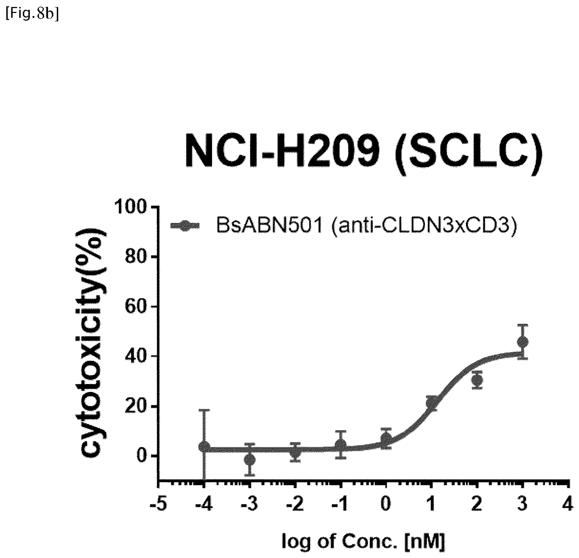

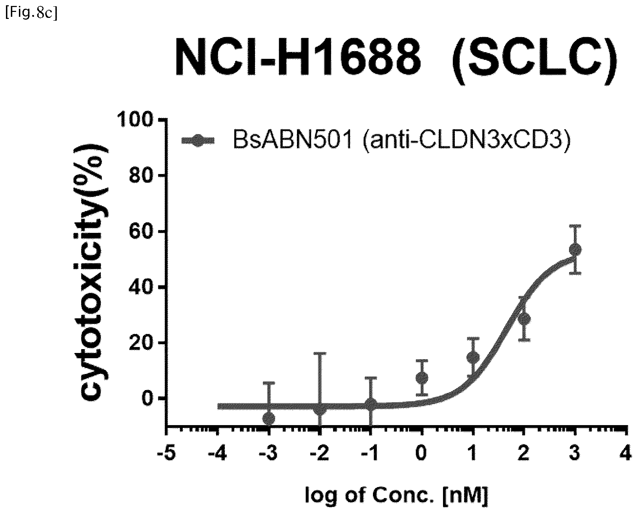

As a result, as shown in Figures 8a to 8c, effective anticancer activity was confirmed in all claudin-3 positive cell lines.

### [Industrial Applicability]

As described above, the present disclosure relates to a composition for preventing or treating small cell lung cancer with reduced side effects, comprising an anti-claudin-3 antibody as an active ingredient. When targeting claudin-3, the ADCC activity of the antibody is significantly higher in cancer cells while being lower in normal cells, and thus, it can be usefully applied to the development of a small cell lung cancer-specific therapeutic agent with fewer side effects.

## Claims

1. A composition for preventing or treating small cell lung cancer with reduced side effects, comprising an anti-claudin-3 antibody as an active ingredient.

2. The composition for preventing or treating small cell lung cancer with reduced side effects according to claim 1, wherein the composition does not exhibit cytotoxic activity against normal cell tissues.

3. The composition for preventing or treating small cell lung cancer with reduced side effects according to claim 1, wherein the side effect is any one selected from the group consisting of leukopenia, lymphopenia, fatigue, anemia, dyspnea, vomiting, stomatitis, cough, and reproductive dysfunction.

4. The composition for preventing or treating small cell lung cancer with reduced side effects according to claim 1, wherein the antibody comprises:
an antibody heavy chain variable region (VH) comprising a heavy chain complementarity determining region (VH-CDR1) comprising the amino acid sequence represented by SEQ ID NO: 1, a heavy chain complementarity determining region (VH-CDR2) comprising the amino acid sequence represented by SEQ ID NO: 2, and a heavy chain complementarity determining region (VH-CDR3) comprising the amino acid sequence represented by SEQ ID NO: 3; and
an antibody light chain variable region (VL) comprising a light chain complementarity determining region (VL-CDR1) comprising the amino acid sequence represented by SEQ ID NO: 4, a light chain complementarity determining region (VL-CDR) comprising the amino acid sequence represented by SEQ ID NO: 5, and a light chain complementarity determining region (VL-CDR) comprising the amino acid sequence represented by SEQ ID NO: 6.

5. The composition for preventing or treating small cell lung cancer with reduced side effects according to claim 1, wherein the antibody is afucosylated.

6. The composition for preventing or treating small cell lung cancer with reduced side effects according to claim 1, wherein the antibody is an anti-claudin-3 and anti-CD3 bispecific antibody.

7. The composition for preventing or treating small cell lung cancer with reduced side effects according to claim 1, wherein the composition is administered to patients with small cell lung cancer who have received chemotherapy drugs.

8. The composition for preventing or treating small cell lung cancer with reduced side effects according to claim 7, wherein the chemotherapy drug is cisplatin, carboplatin, etoposide, topotecan, cyclophosphamide, doxorubicin, vincristine, lurbinectedin, irinotecan, or a combination thereof.

9. A method for providing information for diagnosing small cell lung cancer, comprising measuring the level of claudin-3 protein from a sample of a patient suspected of having small cell lung cancer.

10. Use of an anti-claudin-3 antibody for the manufacture of a pharmaceutical composition for treating small cell lung cancer with reduced side effects.

11. The use of an anti-claudin-3 antibody according to claim 10, wherein the side effect is any one selected from the group consisting of leukopenia, lymphopenia, fatigue, anemia, dyspnea, vomiting, stomatitis, cough, and reproductive dysfunction.

12. The use of an anti-claudin-3 antibody according to claim 10, wherein the antibody comprises:
an antibody heavy chain variable region (VH) comprising a heavy chain complementarity determining region (VH-CDR1) comprising the amino acid sequence represented by SEQ ID NO: 1, a heavy chain complementarity determining region (VH-CDR2) comprising the amino acid sequence represented by SEQ ID NO: 2, and a heavy chain complementarity determining region (VH-CDR3) comprising the amino acid sequence represented by SEQ ID NO: 3; and
an antibody light chain variable region (VL) comprising a light chain complementarity determining region (VL-CDR1) comprising the amino acid sequence represented by SEQ ID NO: 4, a light chain complementarity determining region (VL-CDR) comprising the amino acid sequence represented by SEQ ID NO: 5, and a light chain complementarity determining region (VL-CDR) comprising the amino acid sequence represented by SEQ ID NO: 6.

13. The use of an anti-claudin-3 antibody according to claim 10, wherein the antibody is afucosylated.

14. The use of an anti-claudin-3 antibody according to claim 10, wherein the antibody is an anti-claudin-3 and anti-CD3 bispecific antibody.

15. A method for treating small cell lung cancer with reduced side effects comprising administering an effective amount of a composition comprising an anti-claudin-3 as an active ingredient to a subject in need thereof.

16. The method for treating small cell lung cancer with reduced side effects according to claim 15, wherein the subject is a patient with small cell lung cancer who has received chemotherapy drugs.

17. The method for treating small cell lung cancer with reduced side effects according to claim 16, wherein the chemotherapy drug is cisplatin, carboplatin, etoposide, topotecan, cyclophosphamide, doxorubicin, vincristine, lurbinectedin, irinotecan, or a combination thereof.

18. A method for treating small cell lung cancer comprising administering an effective amount of a composition comprising an anti-claudin-3 as an active ingredient to a patient with small cell lung cancer who has received chemotherapy drugs.

19. The method for treating small cell lung cancer according to claim 18, wherein the chemotherapy drug is cisplatin, carboplatin, etoposide, topotecan, cyclophosphamide, doxorubicin, vincristine, lurbinectedin, irinotecan, or a combination thereof.
